# EUROPEAN PATENT SPECIFICATION

(11) **EP 1 166 108 B1**
(45) Date of publication and mention of the grant of the patent: **12.11.2003**
(21) Application number: 00912653.3
(22) Date of filing: 28.03.2000
(51) Int. Cl.: G01N 33/49

(54) **METHOD FOR THE SEPARATION OF FETAL CELLS FROM THE MATERNAL PERIPHERAL BLOOD**
VERFAHREN ZU TRENNUNG FÖTALER ZELLEN AUS MATERNALEM BLUT
TECHNIQUE PERMETTANT DE SEPARER LES CELLULES FOETALES DU SANG PERIPHERIQUE MATERNEL

(30) Priority: 30.03.1999 IT MI990652
(43) Date of publication of application: 02.01.2002
(73) Proprietor: Sitar, Giammaria, 27020 Carbonara al Ticino (PV) (IT)
(72) Inventor: Sitar, Giammaria, 27020 Carbonara al Ticino (PV) (IT)
(86) International application number: EP0002718
(87) International publication number: WO00060351

(56) References cited:
- US-A- 5 432 054
- US-A- 5 489 386
- US-A- 5 663 051
- US-A- 5 676 849

## Description

### FIELD OF INVENTION

The invention relates to a method and apparatus for the separation of maternal blood and more particularly pertains to the isolation of fetal cells circulating in maternal blood. Cell densities are first modified by transferring maternal blood into non-physiological liquid. Maternal blood is then separated by a single discontinuous density gradient centrifugation step using a previously patented separation device.

### PRIOR ART

A long sought goal of human genetics has been the development of reliable non-invasive procedures for prenatal genetic diagnostic, that do not endanger the fetus and the mother .

Presently prenatal genetic diagnosis is divided in two phases:
a. a non invasive screening test called "triple test" which is offered cost-free by national health authority in many countries to pregnant women aged over 36 years of age or to parents with hereditary genetic diseases. The triple test has a reliability index in which false negative are rare (non elevated risk of encountering an unexpected situation), but where false positive are high (the needless anxiety of the future parents, needless amniocentesis, needles fetal risk, cost for state and families)
b. two invasive tests are suggested to those women positive to the triple tests: either amniocentesis or chorionic villous sampling.(CVC).Amniocentesis is possible after the XIV weeks of gestation and carries a risk of fetal death of 0.5-2% (data of World Health Organization).CVS is possible after the X weeks of gestation and carries a risk of fetal death of 0.5-5% (data of WHO).

Presently more than two million screening tests are performed annually in Europe and as far as eight millions all over the world, to detect common chromosomal abnormalities.

An alternative approach is the use of non-invasive procedure if fetal cells circulating in maternal blood could be isolated in sufficient number for reliable genetic investigation.

Since 1989 fetal DNA has been consistently found in maternal blood by polymerase chain reaction (PCR). Following these studies by molecular biology techniques, the presence of fetal cells in maternal blood has been later confirmed and is now firmly established. Embryonic cells (before XII weeks of gestation) and fetal cells (after XII weeks of gestation) are collectively termed "fetal cells", in the international literature. Several procedures have been proposed in literature to isolate these few cells for non-invasive genetic investigation, but the final fetal cell yield is so low that it precludes reliable cytogenetic analysis by fluorescence in situ hybridization (FISH) or other genetic procedures due to an enormous maternal cell contamination.

The most favorable candidate cell type to be isolated for prenatal non-invasive genetic investigation is the nucleated red blood cell (NRBC), which is exceptionally rare in adult blood while in early fetal blood NRBCs are the most represented cell type together with stem cells.

Fetal white blood cells are present in extremely low percentage in fetal blood during the first trimester of pregnancy, it is therefore highly improbable to find them in maternal blood during the first trimester of pregnancy.

At least 20 fetal cells have to be isolated from maternal blood for reliable genetic investigation. According to the literature, few hundreds fetal cells are circulating in 25 ml maternal peripheral blood,
within 150-200.10⁶ maternal nucleated cells and 100-150. 10⁹RBCs.

This exceedingly low number of fetal cells within a large bulk of maternal cells represents the major obstacle to be overcome especially in view of the fact that a multi-step procedure produces a cellular loss along each step. The preferred method must therefore try to minimize cellular loss and obtain fetal cell isolation by a single-step procedure.

Patents are known disclosing methods for enriching and isolating fetal cells from peripheral maternal blood.

USP 5.676,849 refers to a method for enriching a maternal whole blood sample for desired fetal cell population based on the density gradient centrifugation and passing the desired fraction containing fetal cell population through a counterflow stabilized charge-flow separator apparatus.

USP 5,489386 discloses a density gradient medium for the isolation of rare cells including fetal nucleated erythrocytes from peripheral material blood, said medium comprising a colloidal density gradient medium dispersed in a meltable gel.

USP 5,432,054 refers to a method for isolating and enriching rare cells, including fetal nucleated erythrocytes from peripheral maternal blood, including two centrifugation steps.

The first centrifugation step has the aim of obtaining a red blood cell fraction.

The second centrifugation is carried out in a vessel containing a density gradient medium consisting of a colloid dispersed in a meltable gel.

After hemolysis of maternal red blood cells and melting the gel, the enriched fetal red blood cell fraction is centrifuged through a density gradient medium to obtain a fraction enriched in fetal red blood cells.

Standard cell separation methods to isolate fetal cells from maternal blood use a first step whereby fetal cells are enriched by density gradient centrifugation in standard centrifuge tubes followed by highly sophisticated technology as centrifugal eluitration, fluorescence activated cell sorting or charge flow separation.

Density gradient centrifugation in standard centrifuge tubes produces major cellular loss, at least 50% of cells initially present in the starting cell sample hit the tube walls where they stick or aggregate falling down to the tube bottom), furthermore cell bands which are found layered along the density gradient cannot be recovered without introducing some hydrodynamic disturbances due to manual procedure.

Alternative methods which use the sophisticated and modern technologies above mentioned, beside being expensive and time consuming , are technically demanding and require skillful operators. ("Cell separation: methods and selected applications", Pretlow T.G. and Pretlow T.P. eds. Academic Press, New York, 1982). Also large initial investment for purchasing expensive equipment is required. These drawbacks make them unlikely to become routine tests in prenatal non-invasive genetic investigation. Unlike conventional methods and technologies used for isolating fetal cells from maternal blood, the invention provides for a single centrifugation step in a cell separator device previously patented.

When it is a matter to isolate NRBCs from maternal blood, in a density gradient centrifugation, besides the above mentioned technical problems, the most prominent difficulty to overcome is the fact that the buoyant density distribution profile of NRBCs, lymphocytes and monocytes, does overlap, as illustrated in Table 1 (Haematologica 1997).

| **Type of cell** | **cell density (g/ml)** |
|---|---|
| Lymphocyte | 1.054-1.077 |
| NRBC | 1.065-1.093 |
| Monocyte | 1.058-1.064 |

### SUMMARY OF THE INVENTION

We have discovered a new method which is rapid, simple, unexpensive and above all without any risk for the mother and for the fetus, to isolate fetal cells from maternal blood in sufficient number for reliable non invasive prenatal genetic investigation.

In conclusion the present invention relates to a method to obtain fetal cells from maternal blood, comprising the following steps:
a. maternal blood (25 ml) obtained from an antecubital vein of the arm is transferred into non-physiological tissue culture medium;
b. an aqueous solution of Na citrate, citric acid and dextran is immediately added;
c. maternal blood as diluted in a. and b. is introduced into a separation device, immediately followed by the introduction of a solution having an higher density than maternal blood, containing a RBCs aggregating agent (Ficoll);
d. nucleated cells having a lower density than the liquid introduced below maternal blood in the separation device, are isolated by a single discontinuous density gradient centrifugation;
e. nucleated cells, isolated by the previous step, are washed in phosphate buffered saline, transferred in tissue culture media and placed in a CO₂incubator to regain physiological cell metabolism;
f. fetal cells present in the isolated cell fraction are recognized by appropriate procedure and counted.

### BRIEF DESCRIPTION OF THE DRAWING

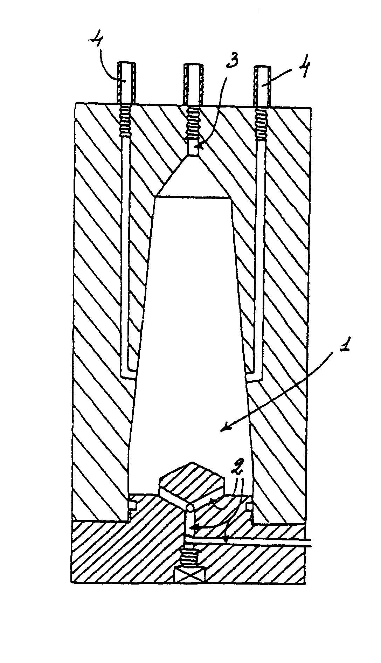

Fig. 1 represents a schematic view of a separation device previously described (PCT/EP98/06865) useful in the easy and very clear collection of cellular fractions which have been separated in the device after centrifugation. The separation device, having a base and a top, comprises an elongated chamber (1), whose cross section decreases from the base towards the device top, at least a first channel (2) one end of which opens onto the inside of the said chamber near the said base, the other end connectable to a pressurized liquid source (not shown), and a second channel (3) one end of which opens into the same chamber (1), corresponding to the device top while the other end opening onto the exterior of the device, and characterized by the fact that in the said device there is at least one additional channel (4), one end of which opens at a middle level of said chamber height, the other end opening onto the exterior of the device .

### DESCRIPTION OF THE INVENTION

The aim of the present invention is to find a method which allows the isolation of fetal cells from maternal peripheral blood in sufficient number for reliable prenatal noninvasive genetic investigation. Standard procedures to isolate fetal NRBCs from maternal blood use density gradient centrifugation as a first step for enrichment of fetal cells. When an heterogeneous cell population is centrifuged in a continuous density gradient, whose density at the bottom is greater than the buoyant density of the different cells present in the sample, then cells will float to positions in the gradient where density of the solution is identical with that of the cell, provided the product of the time and centrifugal field exceeds a certain value. After that no further sedimentation will occur, independently of the time of centrifugation. A distinct advantage contributing to the widespread use of density gradient centrifugation is that a very large number of cells can be simultaneously fractionated. When the density distribution profile of different cell populations does overlap, their separation by density gradient centrifugation is impossible, as it is the case for NRBCs, lymphocytes and monocytes. The above mentioned aim is satisfied by modifying the density of cells present in maternal blood, more particularly that of NRBCs, lymphocytes and monocytes, and is obtained transferring maternal blood into non-physiological culture medium. When maternal blood is transferred into non-physiological culture medium, NRBCs density decreases while cell density of lymphocytes and monocytes increases. Said non-physiological culture medium is the following:

| | |
|---|---|
| pH | 6.4-6.6 |
| osmolality | 300-330 mOsm |
| Na⁺ | 150-170 mmol/l |
| K⁺ | 4.5-5.5 mmol/l |
| Cl⁻ | 100-115 mmol/l |
| Ca⁺⁺ | 1.00-2.50 mmol/l |
| glucose | 400-500 mg/dl |
| lactate | 10-20 mg/d |

The reason why cell densities of desired blood cells change, rendering possible their separation according to the present invention is not clearly understood and represents a surprising feature of the present invention. An additional choice is between continuous or discontinuous density gradient. In continuous gradient sedimented cells will be distributed according to their actual densities, while in discontinuous gradients cells are concentrated at interfacies and this may be a convenient way of compressing together, for preparative purposes, certain segments of the cell distribution observed in continuous gradients. By introducing a discontinuous density step, in the density gradient centrifugation, most NRBCs float at the interface with separating medium, being lighter, while most lymphocytes and monocytes sink down to the bottom of the separation device, being heavier than the separating medium.

Fetal isolated cells are mainly NRBCs, but also some fetal stem cells are present in the isolated cellular fraction. RBCs present in maternal blood are aggregated by the aggregating agent present in the separating medium introduced right below maternal blood into the separation device and fall down to the bottom of the separation device. Completing the isolation of NRBCs with a single step procedure does greatly increase cell yield of isolated fetal cells useful for genetic investigation, since cells are not lost in additional separation steps.

Several separation devices could be used for the aim of the present invention, between them the separation devices described in patent US 4.424.132 (Asahi Kasei Kogyo Kabushiki Kaisha), in patent GB 2.075.376 (Institute of Medical Sciences), in patent FR 2 350 885 (Baxter Travenol Laboratorie) and in PCT/EP98/06865 (Giammaria Sitar, inventor and applicant).

Between these separation devices, that described in PCT/EP98/06865 and shown in Fig.1, is the preferred one.

After discontinuous density gradient separation, the isolated cellular fraction, containing fetal NRBCs and fetal stem cells within a larger maternal cell population, is washed and transferred into physiological tissue culture medium to regain physiological cell metabolism. After 24hours in vitro culture, in a CO₂ incubator, cells are investigated by appropriate procedures to ascertain the presence and the number of fetal cells. The presence of ε-chain Hemoglobin distinguishes fetal from maternal NRBCs, since ε-chain hemoglobin is synthesized only in early fetal life till the 12^{th-} 14^{th}weeks of gestation. Maternal NRBCs express only α and β-chains of hemoglobin. γ-chains of hemoglobin could be found both in fetal and in maternal NRBCs, therefore their presence has not been considered.

Additionally fetal cells have been searched for by FISH using fluorescent probes for Y chromosome, which , of course, is absent in maternal cells, which are all XX. The presence of cells with an XY pattern of sex chromosomes is therefore compelling evidence of the fetal origin of such cells.

The procedure disclosed by said invention allows prenatal noninvasive genetic investigation to be completed in short time and very early during pregnancy (as early as the 8^{th} week of gestation). The most prominent advantage of said procedure is that it doesn't carry any risk for the mother or the fetus, since it only requires 25 ml maternal peripheral blood.

### EXAMPLE

25 ml of maternal blood, anticoagulated with heparin, is transferred into a 50-ml tissue culture flask containing 25 ml of tissue culture medium having the composition detailed in Table 2.

**TABLE 2**

| **Component** | **g/l** |
|---|---|
| | |
| calcium chloride 2H₂O | 0.265 |
| ferric nitrate 9H₂O | 0.00072 |
| magnesium sulfate | 0.09767 |
| potassium chloride | 0.4 |
| sodium acetate | 0.05 |
| sodium chloride | 6.8 |
| sodium phosphate monobasic | 0.122 |
| DL-alanine | 0.05 |
| L-arginine-HCl | 0.07 |
| DL-aspartic acid | 0.06 |
| L-cysteine-HCl-H₂O | 0.00011 |
| L-cysteine 2HCI | 0.026 |
| DL-glutamic acid | 0.1336 |
| L-glutamine | 0.1 |
| glycine | 0.05 |
| L-histidine-HCl-H₂O | 0.02188 |
| hydroxyl-L-proline | 0.01 |
| DL-isoleucine | 0.04 |
| DL-leucine | 0.12 |
| L-lysine-HCl | 0.07 |
| DL-methionine | 0.03 |
| DL-phenylalanine | 0.05 |
| L-proline | 0.04 |
| DL-serine | 0.05 |
| DL-threonine | 0.06 |
| DL-tryptophan | 0.02 |
| L-tyrosine 2Na-2H₂O | 0.05766 |
| | |
| DL-valine | 0.05 |
| ascorbic acid-Na | 0.0000566 |
| D-biotin | 0.00001 |
| calciferol | 0.0001 |
| choline chloride | 0.0005 |
| folic acid | 0.00001 |
| menadione (sodium bisulfite) | 0.000016 |
| myo.inositol | 0.00005 |
| niacinamide | 0.000025 |
| nicotinic acid | 0.000025 |
| p-amino benzoic acid | 0.00005 |
| D-pantothenic acid (hemicalcium) | 0.00001 |
| pyridoxal-HCL | 0.000025 |
| pyridoxine-HCI | 0.000025 |
| retinol aceate | 0.00014 |
| riboflavin | 0.00001 |
| DL-α-tocopherol phosphate-Na | 0.00001 |
| Thiamine-HCI | 0.00001 |

Immediately after, 5 ml of an acqueus solution are added, containing citric acid 1g/125 ml, Na citrate 2.25 g/125ml and dextran 3g/125 ml, thus obtaining the following non-physiological conditions

| | |
|---|---|
| pH | 6.5 |
| Osmolality | 320 mOsm/l |
| Na | 165 mmol/l |
| K | 5.35 mmol/l |
| Cl | 110 mmol/l |
| Ca | 1.25 mmol/l |
| glucose | 500 mg/l |
| lactate | 10 mg/dl |

Maternal blood, diluted as above, is introduced into the separation device (Fig.1) from the bottom, through channel 2, followed by 45ml
of a Ficoll-Na-diatrizoate solution having a density of 1.068 g/ml and an osmolality of 290 mOsm/l. This latter solution uses Ficoll as RBCs aggregating agent and Nadiatrizoate to obtain the desired density, dissolved in Tris-HCl and Tris-saline balanced salt solution.

By proceding in the said manner, maternal blood occupies the upper part of the elongated chamber, in the separation device, down to the level of the outlet ports of lateral channels (4), below which is the separating media (Ficoll-Na-diatrizoate solution). The separation device, thus loaded, is then centrifuged for 1 hour, at 400 gravity, to obtain successive stratification of the different cellular populations initially mixed in the starting maternal blood.

RBCs, aggregated by Ficoll, sediment to the bottom of the elongated chamber, above them are present the great majority of white blood cells (neutrophils, basophils, eosinophils, monocytes and lymphocytes) whose density is greater than the density of the separating medium. The low density cell fraction (density <1.068g/ml)floating at the interface between plasma and the separating medium, contains most, if not all, NRBCs, present in the starting maternal blood sample, together with some stem cells, lymphocytes and monocytes.

For the separate collection of this low density cell fraction, enriched in fetal cells, a heavy water-immiscible liquid (Fluorinert ® FC-43, 3M Company), is introduced at the base , through channel 2, at a flow velocity of 2ml/min, while simultaneously pumping air from the top, through channel 3, at the same rate.

Thus the low density cellular fraction floating at the level of the lateral channels (4)exit holes, located half way in the elongated chamber, is compressed and forced out through these and is collected in 5 minutes without remixing with other cellular fractions because of hydrodynamic disturbances. The cellular fraction thus obtained is washed in tissue culture medium and nucleated cells are counted. Differential count of cell types present is obtained on cytocentrifuged cell preparations, stained with May-Grunwald and Giemsa.

Identification of fetal NRBCs was carried out by immunostaining for embrionic hemoglobin and by FISH. Immunostaining for embrionic hemoglobin was performed using anti-ε chains Hb monoclonal antibody and FISH was performed with X and Y specific probe mixture (Vysis Inc, IL, USA). Results obtained in the isolation of NRBCs from maternal blood in 12 different samples are shown in Table 3.

The invention being thus described, various modifications will occur to those skilled in the art, to modify cell density and thus separate cells having similar densities in physiological conditions, and all such variations are intended to be included in the scope of the invention as defined by the following claims.

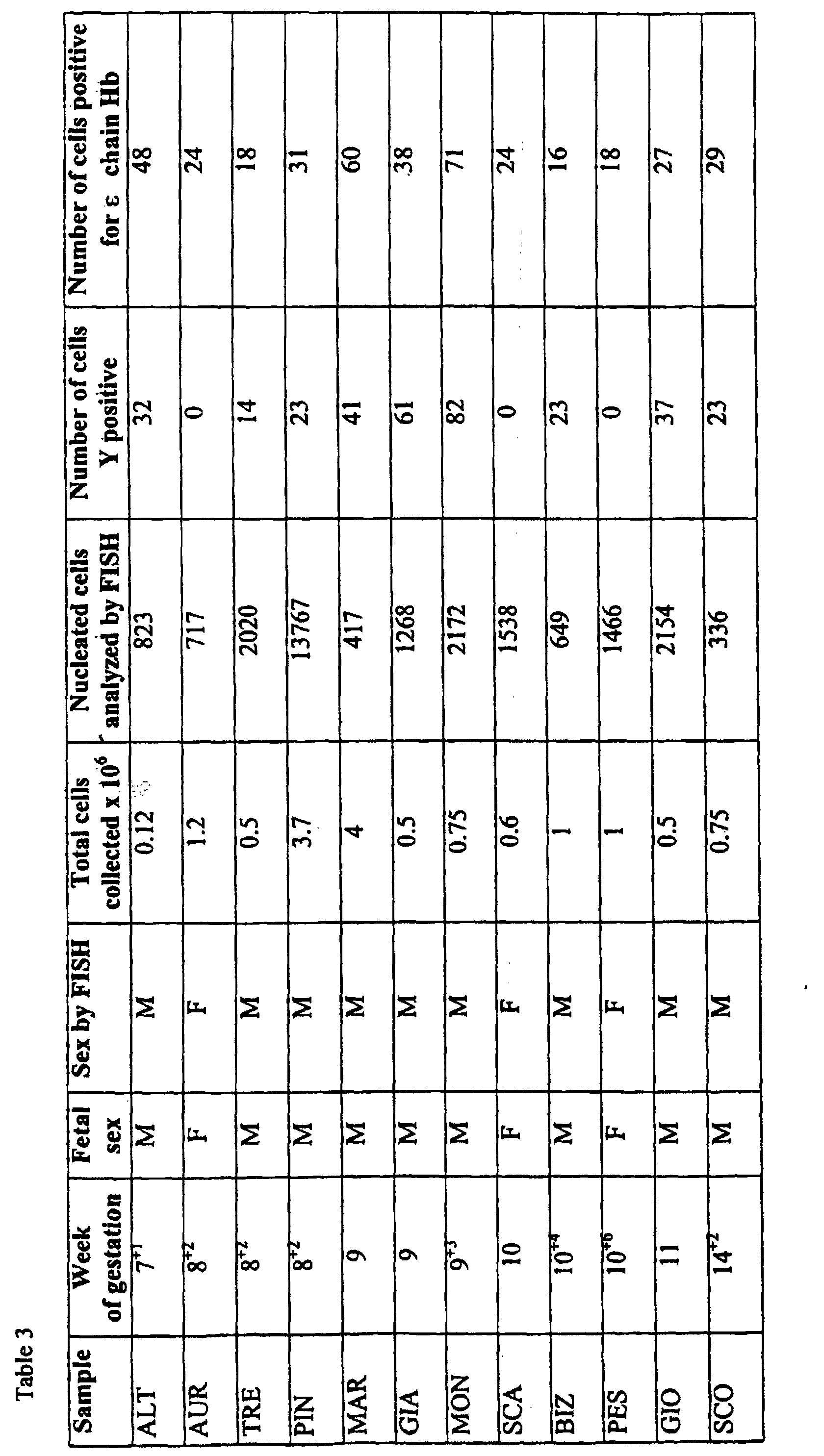

## Claims

1. A method for isolating fetal cells present in maternal peripheral blood for prenatal genetic investigation, comprising the steps of:
a) transferring maternal blood into non-physiological tissue culture medium, which after addition of an aqueous solution containing citric acid, Na citrate and dextran, has the following characteristics:
| | |
|---|---|
| pH | 6.4-6.6 |
| osmolality | 300-330 mOsm |
| Na⁺ | 150-170 mmol/l |
| K⁺ | 4.5-5.5 mmol/l |
| Cl⁻ | 100-115 mmol/l |
| Ca⁺⁺ | 1.00-2.50 mmol/l |
| glucose | 400-500 mg/dl |
| lactate | 10-20 mg/d |
b) maternal blood, as modified in a) is transferred into a cell separation device, followed by the introduction into the said separation device of a liquid having an higher density and containing a RBCs aggregating agent;
c) the nucleated cells, having a lower density than the liquid introduced in the step b) are isolated, in the discontinuous density gradient, by subjecting the separation device to centrifugal force;
d) the isolated cells are washed and resuspended in tissue culture medium to regain physiological cell metabolism;
e) fetal cells are identified by appropriate procedures and counted.

2. The method of claim 1 whereby fetal NRBCs are isolated.

3. The method of claim 1 in which the non-physiological medium obtained in step a) has the following characteristics:
| | | |
|---|---|---|
| pH | 6.5 | |
| osmolality | 320 | mOsm |
| Na⁺ | 165 | mmol/l |
| K⁺ | 5.35 | mmol/l |
| Cl⁻ | 110 | mmol/l |
| Ca⁺⁺ | 1.25 | mmol/l |
| glucose | 500 | mg/dl |
| lactate | 10 | mg/dl |

4. The method of claim 1 in which the RBCs aggregating agent of step b) is Ficoll.

5. The method of claim 1 in which the density of the liquid introduced in the separation device by the step b) is 1.068 g/ml.

6. The method of claim 1 in which the separation device used in step b), comprises an elongated chamber (1), whose cross section decreases from the base towards the top, at least a first channel (2) one end of which opens into the said chamber near the said base and the other end is connected to a pressurized liquid source, and a second channel (3) one end of which opens into the same chamber (1) at the device top while the other end opens at the exterior of the device, the said device further comprising at least one additional channel (4), one end of which opens at a middle level of said chamber height and the other end opens at the exterior of the device.

## Patentansprüche

1. Ein Verfahren zur Isolierung von im periphärischen Mutterblut vorhandenen Phötuszellen für eine vorgeburtliche genetische Untersuchung, umfassend folgende Schritte:
a) Übertragung des Mutterblutes in ein nicht-physiologisches Gewebe-Kulturmittel, das nach Zusatz einer wässrigen, Zitronensäure, Natriumzitrat und Dextran enthaltenden Lösung folgende Eigenschaften aufweist:
| | | |
|---|---|---|
| pH | 6,4-6,6 | |
| Osmolalität | 300-330 | mOsm/L |
| Na⁺ | 150-170 | mMol/L |
| K⁺ | 4,5-5,5 | mMol/L |
| Cl⁻ | 100-115 | mMol/L |
| Ca⁺⁺ | 1,00-2,50 | mMol/L |
| Glukose | 400 - 500 | mg/Dl |
| Laktat | 10-20 | mg/Dl |
b) Das wie in a) veränderte Mutterblut wird in eine geeignete Zell-Trennungsvorrichtung übertragen, gefolgt von der Einführung in die genannte Zell-Trennungsvorrichtung einer Flüssigkeit höherer Dichte, die ein Erythrozyten-Aggregationsmittel enthält,
c) Die Kernzellen, die eine niedrigere Dichte als die im Schritt b) eingeführte Flüssigkeit aufweisen, werden in einem diskontinuierlichem Dichtegradienten dadurch isoliert, dass die Zell-Trennungsvorrichtung einer Zentrifugalkraft unterworfen wird;
d) Die isolierten Zellen werden gewaschen und in einem Gewebe-Kulturmittel wieder suspendiert, um ihren physiologischen Zellenstoffwechsel wiederzugewinnen;
e) Die Phötuszellen werden durch geeignete Verfahren **gekennzeichnet** und gezählt.

2. Das Verfahren nach Anspruch 1, wodurch fötale nukleierte-Erythroziten isoliert werden.

3. Das Verfahren nach Anspruch 1, in dem das im Schritt a) erhaltene nichtphysiologische Medium folgende Eigenschaften aulweist:
| | | |
|---|---|---|
| pH | 6,5 | |
| Osmolalität | 320 | mOsm/L |
| Na⁺ | 165 | mMol/L |
| K⁺ | 5,35 | mMol/L |
| Cl⁻ | 110 | mMol/L |
| Ca⁺⁺ | 1,25 | mMol/L |
| Glukose | 500 | mg/Dl |
| Laktat | 10 | mg/Dl |

4. Das Verfahren nach Anspruch 1, in dem das Erythroziten-Aggregationsmittel im Schritt b) Ficoll ist.

5. Das Verfahren nach Anspruch 1, in dem die Dichte der im Schritt b) in die Zell-Trennungsvorrichtung eingeführten Flüssigkeit 1,068 g/ml ist.

6. Das Verfahren nach Anspruch 1, in dem die im Schritt b) verwendete Zell-Trennungsvorrichtung eine verlängerte Kammer (1) umfasst, deren Querschnitt vom Boden- zum Kopfteil hin abnimmt, mit mindestens ein erster Kanal (2), dessen eines Ende in die genannte Kammer in der Nähe des genannten Bodenteils mündet und dessen anderes Ende mit einer Quelle von Druckflüssigkeit verbunden ist, und einem zweiten Kanal (3), dessen eines Ende in den Kopfteil derselben Kammer (1) mündet, während das andere Ende sich an der Außenseite der Vorrichtung öffnet, wobei die genannte Vorrichtung noch mindestens einen weiteren Kanal (4) umfasst, dessen eines Ende sich an einem mittleren Höhenniveau der genannten Kammer öffnet, und dessen anderes Ende sich an der Außenseite der Vorrichtung öffnet.

## Revendications

1. Procédure permettant d'isoler les cellules foetales présentes dans le sang périphérique maternel afin de pouvoir effectuer l'analyse génétique prénatale. Cette méthode comprend les phases suivantes :
a) Le sang maternel est ajouté à un terrain de culture non physiologique qui, après l'ajout d'une solution aqueuse contenant de l'acide citrique, Na citrate et dextrane, présente les caractéristiques suivantes :
| | |
|---|---|
| pH | 6.4-6.6 |
| osmolalité | 300-330 mOsm |
| Na⁺ | 150-170 mmol/l |
| K⁺ | 4.5-5.5 mmol/l |
| Cl⁻ | 100-115 mmol/l |
| Ca⁺⁺ | 1.00-2.50 mol/l |
| Glucose | 400-500 mg/dl |
| Lactate | 10-20 mg/dl |
b) Le sang maternel, modifié selon les indications du paragraphe a) est introduit dans un appareil séparateur approprié, puis un liquide présentant une plus forte densité et contenant un agent d'agrégation des globules rouges est introduit dans cet appareil.
c) Les cellules nucléées qui ont une densité inférieure à celle du liquide introduit au cours de la phase b) sont isolées, dans le gradient de densité discontinu, sous l'effet de la centrifugation de l'appareil séparateur.
d) Les cellules isolées sont lavées et resuspendues dans un terrain de culture, de façon à rétablir un métabolisme cellulaire physiologique.
e) Les cellules foetales sont identifiées selon des procédures appropriées et comptées.

2. Procédure selon la revendication 1 au cours de laquelle les érythroblastes foetaux sont isolés.

3. Procédure selon la revendication 1 au cours de laquelle le terrain de culture non physiologique obtenu lors de la phase a), présente les caractéristiques suivantes :
| | | |
|---|---|---|
| pH | 6.5 | |
| osmolalité | 320 | mOsm |
| Na⁺ | 165 | mmol/l |
| K⁺ | 5.35 | mmol/l |
| Cl⁻ | 110 | mmol/l |
| Ca⁺⁺ | 1.25 | mmol/l |
| Glucose | 500 | mg/dl |
| Lactate | 10 | mg/dl |

4. Procédure selon la revendication 1 au cours de laquelle l'agent d'agrégation de la phase b) est le Ficoll

5. Procédure selon la revendication 1 au cours de laquelle la densité du liquide introduit dans l'appareil séparateur lors de la phase b) est de 1,068 g/ml.

6. Procédure selon la revendication 1 au cours de laquelle l'appareil séparateur utilisé lors de la phase b) comprend une cavité de forme élongée (1) dont la section transversale diminue de la base vers le sommet, au moins un premier canal (2) dont l'une des extrémités s'achève dans la cavité élongée située en bas de cette dernière, tandis que l'autre extrémité est raccordée à une source de liquide pressurisée, et un deuxième canal (3) dont l'extrémité s'achève dans la partie supérieure de la cavité de forme élongée, tandis que l'autre extrémité s'achève à l'extérieur de l'appareil séparateur, lequel comprend également au moins un canal supplémentaire (4) dont l'une des extrémités aboutit au niveau de la portion médiane de la cavité, tandis que l'autre extrémité aboutit à l'extérieur de l'appareil séparateur.
